# EUROPEAN PATENT APPLICATION

(11) **EP 3 069 753 A1**
(43) Date of publication of application: **21.09.2016**
(21) Application number: 16157861.2
(22) Date of filing: 29.02.2016
(51) Int. Cl.: A61N 1/372, A61N 1/375

(54) **IMPLANTABLE MEDICAL DEVICE AND MANUFACTURING METHOD THEREFOR**

(30) Priority: 17.03.2015 US 201562134027 P
(71) Applicant: BIOTRONIK SE & Co. KG, 12359 Berlin (DE)
(72) Inventor: Klenner, Rolf, 14554 Seddiner See (DE); Austin, Eric, Portland, OR 97221 (US); Frank, Ian Joseph, Wilsonville, OR 97070 (US); Koch, Sascha, Portland, OR 97225 (US); Hartmann-Bax, Kathy, 14947 Nuthe-Urstromtal (DE)
(74) Representative: Galander, Marcus

(57) **Abstract**

The invention refers to an implantable medical device comprising an housing (60), an electrical circuit unit (20), and a feedthrough component (30), which is electrically connected to at least one contact of the electrical circuit unit, wherein the device further comprises a support (10) fixedly connected to the electrical circuit unit (20). In order to reduce the number of steps during manufacturing in particular for device with a sleeve-type housing the support (10) provides at least one fixture element (13) adapted for a mechanical fixing of the feedthrough component (30) relative to the electrical circuit unit (20) within the housing (60). The invention further refers to a respective manufacturing method.

## Description

The present invention relates to an implantable medical device comprising a housing, an electrical circuit unit, and a feedthrough component which is electrically connected with at least one contact of the electrical circuit unit. The invention further relates to manufacturing method for such an implantable medical device.

Implantable medical devices for providing electrical stimulation to body tissues, for monitoring physiologic conditions, and for providing alternative treatments to drugs are well-known in the art. Exemplary implantable medical devices include implantable cardio defibrillators, (leadless) pacemakers, implantable monitoring devices or sensors, and programmable neuro-stimulator pulse generators. The medical devices typically incorporate a power source connected with an electronic circuit unit having a circuit board. Often implantable medical devices are electrically connected with electrodes which are typically used to test the device and to provide electrical stimuli and/or electrical sensing within the body in which the device is implanted, for example produce stimulation impulses or defibrillator shocks. Alternatively, electronic potentials of body locations are determined. Therefore, an electric connection between the electronic circuit unit within the housing and the electrodes is necessary. Therefore, one or more feedthrough components are provided which permit electrical communication to and from the electrical components and circuitry of the electrical circuit unit contained within the housing while at the same time maintaining the hermeticity of the device. The feedthrough component may be integrated in a header of the implantable medical device.

Document US 6,931,284 B2 discloses an implantable medical device that includes an enclosure or housing having an upper half, a lower half and electrical feedthroughs to which the wires are connected. The known device further contains an energy source, typically a battery, and an electrical module with electrical circuitry containing numerous interconnected electrical components mounted on a circuit board. The device further comprises a support structure for the electrical circuitry. The support structure accommodates an antenna coil, wherein electrical contacts are provided on the support structure to permit electrical coupling to appropriate portions of contacts on circuit board and to the antenna coil. During manufacturing the antenna coil is attached to the support structure. The coil is fixed at the support structure by an adhesive, for example a durable epoxy. Now the electrical module with the circuit board and electrical components is accommodated above the coil so that an adhesive provided over the coil and support structure is in contact with the module. The known device has a complicated design with a lot of parts. This is cost-intensive during manufacturing.

Document US 2009/0192573 A1 refers to a coupling element for mechanical and electrical coupling of a passive and/or active electrical unit to an electrical circuit. The known coupling element is a basically ring-shaped structure comprising a first side and a second side. The first side faces towards a passive and/or active electrical unit and the second side faces toward an electrical circuit unit. The coupling element further comprises at least one electrical contact element configured for electrical contact of the passive and/or active electrical unit with the electrical circuit unit, for example solderable contacts having a rectangular shape. Further, attached to the second side at least one means for secure mechanical connection to the electrical circuit unit is fixedly attached, for example the solderable element is composed in such a way that a solder contact may be produced using SMT-processes. The known coupling element provides a support for the electrical circuit unit, which can be pre-assembled in simplified manner, but does not ease device manufacturing, or reduce the amount of parts necessary for an implantable medical device.

As explained with regard to document US 6,931,284 B2 an implantable medical device with a two-part enclosure eases manufacturing but needs a lot of process steps and parts for manufacturing of the device. In case that a single part sleeve-type can is used for the housing, the manufacturing of the implantable medical device needs to be changed, in particular with regard to the mounting of the feedthrough component.

Hence, it is an object of the present invention to provide an implantable medical device, in particular with a sleeve-type can, that can be manufactured with a reduced number of steps and still provides maximum reliability. Further, the object of the present invention consists in providing a manufacturing method with reduced cost.

The above task is solved by an implantable medical device with the features of claim 1. In particular the device further comprises a support fixedly connected to the electrical circuit unit, wherein the support provides at least one fixture element adapted for a mechanical fixing of the feedthrough component relative to the electrical circuit unit within the housing.

The above inventive implantable medical device is based on the finding that it is necessary to attach and thereby align the feedthrough component relative to the electrical circuit unit prior to inserting the electrical circuit unit into the housing which is preferably a sleeve-type can. It was observed that limited access during the module insertion renders an in-situ manipulation for the alignment of the feedthrough component to the housing impractical. The inventive solution has the further advantage that it allows usage of not only rigid electrical circuit units but also of high-density interconnect flex substrates. This has the further advantage that a lower device profile is achieved.

In the present invention the electrical circuit unit comprises an electronic circuit with active and/or passive electrical components within a (functioning) configuration produced by electrical current flowing through the components in a closed loop. The electrical circuit unit further comprises a circuit board on which the electrical components and their electrical interconnection are provided, wherein the circuit board is also referred to as a printed wiring board (PWB), printed circuit board (PCB), or etched wiring board (EWB) used for the mechanical fastening and the electrical connection of electronic components.

Preferably, the support has a lightweight plate-like or frame-like structure which has a small thickness.

In a preferred embodiment the at least one fixture element is a form-fit element, for example a staking pin cooperating with a respective form-fit element, for example a through going opening, of a bracket of the feedthrough component. The staking pin provided at the support cooperates with the through going opening of the feedthrough component so that the feedthrough component is easily attached to and aligned with the support and thereby with the electrical circuit unit. Alternatively, the bracket of feedthrough component comprises the staking pin and the support the through going opening. Preferably, the axis of the staking pin and hence the axis of the through going opening runs into a direction essentially perpendicular to the extension of the support and/or the electrical circuit unit. The staking pin and the through going opening may have a circular, a rectangular, a square or another suitable cross-section. For proper alignment the support and/or the feedthrough component (or its bracket) comprises at least two fixture elements (for example two staking pins or two respective through going openings).

The bracket of the feedthrough component is formed such that it preferably provides a weld shield. In one example the weld-stop is realized by a specific material or specific materials or specific material compositions; in one example it is realized by a specific geometry or specific geometries; or it is established a combination of both specific material or materials and geometry or geometries.
In case of the specific material or the specific materials or the specific material compositions, the bracket may comprise these material or materials or material compositions. In other embodiments, these material/materials/material composition may be incorporated in the bracket, plated, coated on the bracket or otherwise applied to the bracket. In one exemplary embodiment these materials or material compositions are solely located on the bracket location, which is situated between the vent hole in the medical device housing and the electrical circuit unit. One preferred material is a metallic material, for example titanium. Other suitable materials - also suitable non-metallic materials - are possible.
The geometry of the bracket in all dimensions (width, depth or height) may be adapted in any suitable manner to prevent unintended welding. Thereby, the weld shield extends into the same unused space as the staking pins and thereby also performs the task of securing and aligning the feedthrough component in addition to that of the weld shield. The alignment bracket thereby realizes two functions, namely the weld shield and the alignment and mechanical connection with/to the support and thereby with/to the electronic circuit unit.

Preferably, the support is composed of an electrically isolating material, for example polymeric materials, which are unreactive and inert and which are highly resistant to heat, for example Liquide Crystal Polymer (LCP).

It is further of advantage if the fixture element is accommodated in the clearance between the electrical circuit unit and the housing parallel to the feedthrough component because thereby the fixture element makes use of space that would otherwise not have been used owing to the need for clearance between the electrical circuit and the housing.

In another preferred embodiment the support is formed as a frame. Preferably the support is adapted to carry a passive electronic module such as an antenna. Therein, the antenna is preferably an air coil antenna. The frame may be preferably of a rectangular form providing the passive electronic module on one side of the frame. Additionally, the support further provides at least one electrical contact for the passive electronic component on its surface.

In another preferred embodiment, the bracket of the feedthrough component comprises an L-form, wherein a first arm of the L runs perpendicular to the extension of the electrical circuit unit and a second arm of the L comprises the form-fit element, e.g. the through going opening, and runs parallel to the extension of the electrical circuit unit. The bracket formed as an L can easily connect both parts without abusing any additional space in the implant. Further, this configuration allows for a single axis feedthrough attach prior to insertion into the housing. That means, that the bracket attach, as well as the flex band attach, are on the same axis. In another configuration the first and/or second arm with the form-fit element could also be perpendicular to the extension of the circuit unit. The staking pins would have to face forward in the same direction as the feedthrough pins. In that case, the axis for assembling the feedthrough bracket has to be changed, but the bracket component will be easier to manufacture.

For reliable electric connection between the feedthrough component and the electrical circuit unit the electrical circuit unit preferably comprises an interconnect weld ribbon and the feedthrough component comprises at least one tab, preferably a tab array.

In an alternative embodiment, the electrical circuit unit comprises a flex arm extension for direct electric connection with the feedthrough component. This solution saves overall space and additional frees up area on the electrical circuit unit for electrical components on the module.

The above task is further solved by a manufacturing method for an implantable medical device comprising the following steps:
- fixedly connecting the electrical circuit unit with the support, preferably by commonly known solder attaching,
- mechanical fixing of the feedthrough component relative to the electrical circuit unit by means of at least one fixture element provided at the support,
- forming an electrical connection of the feedthrough component with at least one contact of the electrical circuit unit,
- placing the assembly comprising the support with the electrical circuit unit and the feedthrough component within the housing and hermetically sealing the housing.

The above inventive method provides on the one hand a reliable mechanical connection of the feedthrough component to the electronic circuit unit via the support and on the other hand the electric connection of the feedthrough component to the electrical circuit unit. Further, by means of the pre-alignment of the feedthrough component with regard to the electrical circuit unit the assembly process is simplified and the overall amount of process steps is reduced.

In a preferred embodiment of the inventive method for mechanical fixing of the feedthrough component relative to the electrical circuit unit by means of the at least one fixture element provided at the support at first the fixture element of the support is form-fit connected to a corresponding fixture element at the feedthrough component and preferably thereafter fixed by a material engagement, preferably using the corresponding fixture elements. For example the form-fit connection is provided by a staking pin of the support and a through going opening at a bracket of the feedthrough component. Alternatively, the bracket of the feedthrough component may provide a staking pin and the support may provide a respective through going opening. Further preferred the material engagement is provided by molding of the pin over the bracket or the support by means of a heat staking process.

In another preferred embodiment, prior to the connection of the electrical circuit unit to the support a passive electronic module, preferably an antenna, is attached to the support. The passive electronic module may be attached to the support by means of an adhesive. Further, the support may provide contacts for electric connection of the passive electronic module with an electric conducting connector and thereby to the electrical circuit unit.

A full and enabling disclosure of the present invention, including the best mode thereof, directed to one of ordinary skill in the art is set forth in the following specification of the preferred embodiments. Thereby, further features and advantages are presented that are part of the present invention independently of the features mentioned in the dependent claims.

The specification refers to accompanying figures showing schematically
- Fig. 1: a support of a first embodiment of an inventive implantable medical device in a perspective view,
- Fig. 2: an assembly comprising the support of Fig. 1 and an electrical circuit unit in a perspective view,
- Fig. 3: a feedthrough component of the first embodiment of an inventive implantable medical device in a perspective view,
- Fig. 4 and 5: an assembly containing the assembly of Fig. 2 and the feedthrough component of Fig. 3 shown partly in a perspective view (Fig. 4) and partly in a top view (Fig. 5),
- Fig. 6: a perspective exploded view of the first embodiment of an implantable medical device,
- Fig. 7: a battery flange of the first embodiment of an implantable medical device in a perspective view,
- Figs. 8 and 9: an assembly comprising the flange of Fig. 7 and a battery within its housing of the first embodiment of an implantable medical device in two perspective views,
- Figs. 10 and 11: a mechanical connection of the support with the assembly of Figs. 8 and 9 in a perspective view and in a top view,
- Fig. 12: a connection of the housing with the assembly of Figs. 8 and 9 partly in a perspective view, and
- Figs. 13 and 14: the assembled implantable medical device with plug in a side view and a top view.

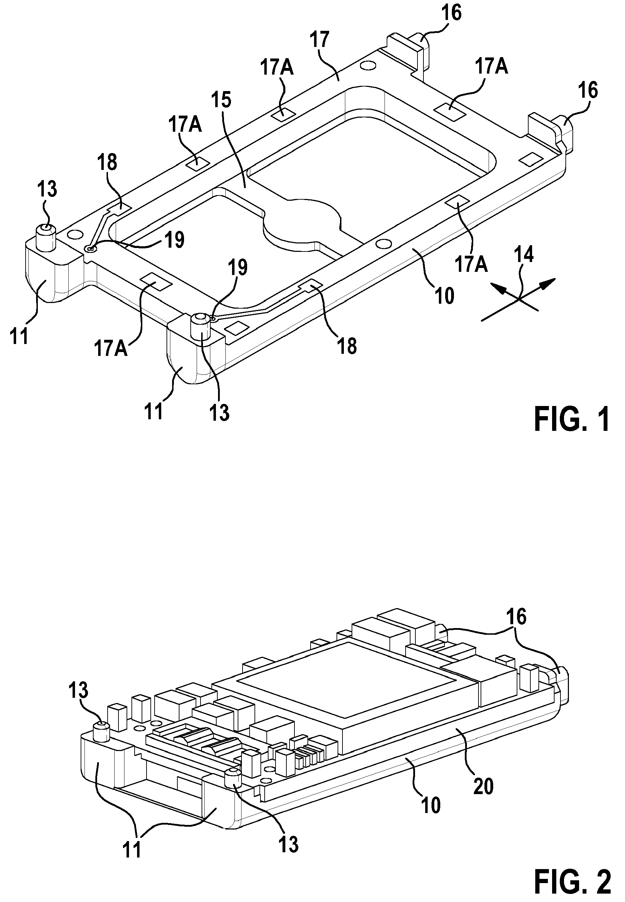

Fig. 1 shows a support 10 which is used to fixedly connect an electrical circuit unit for an implantable medical device such as a leadless pacemaker. The support 10 has a flat rectangular frame-like shape with two noses 11 accommodated on one shorter front side of the rectangle. Each nose 11 comprises one staking pin 13, wherein each staking pin 13 has a circular cross-section and a slightly pointed end. The axis of each staking pin 13 is perpendicular to the extension (see arrows 14) of the rectangular frame of the support 10. The frame of the support 10 further comprises in the centered section a stiffening rib 15. At the shorter side of the support 10 facing the side with the noses 11, two battery tabs 16 are provided pointing in a direction parallel to the extension (see arrows 14) of the rectangular support 10. Further, support 10 comprises a printed circuit board (PCB) construction or interposer 17 for an electrical and mechanical connection of the support 10 with the a electrical circuit unit 20. The interposer 17 is attached to the support 10 by means of solder or adhesive or both.

For the fixed mechanical connection with the electrical circuit unit 20 the interposer 17 of the support 10 provides on its upper surface solderable pads 17A. Further, contact elements 18 are provided on the interposer 17, which are electrically connected to at least one via 19 provided in the interposer 17. Both are used to lead through electrical signal from the upper side side of the interposer 17 (where the contact elements 18 are located) to the otherside, for example to provide electrical contact from electrical circuit unit 20 to the antenna (not shown).

As indicated in Fig. 2 the electrical circuit unit 20 comprises a printed circuit board (PCB) and electrical components such as active and/or passive electrical units attached to the printed circuit board. The electrical circuit unit 20 is fixed to the support 10 via the interposer 17, for example by an adhesive or solder or both. In one embodiment, the electrical circuit unit 20 is fixedly attached to the solderable pads 17A of the interposer 17 and thereby establishing a fixed attachment.

In an embodiment between the electrical circuit unit 20 and the support 10 an antenna (not shown) is accommodated, wherein each end of the coil of the antenna is electrically connected to one of the contact elements 18. In this embodiment the electrical circuit unit 20 is fixedly connected to the antenna and the antenna is fixedly connected to the support 10.

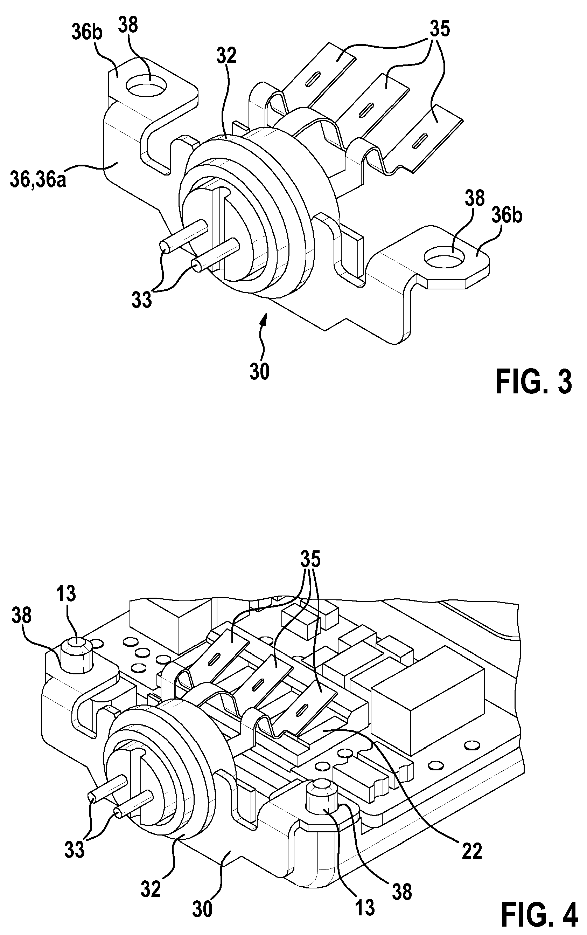

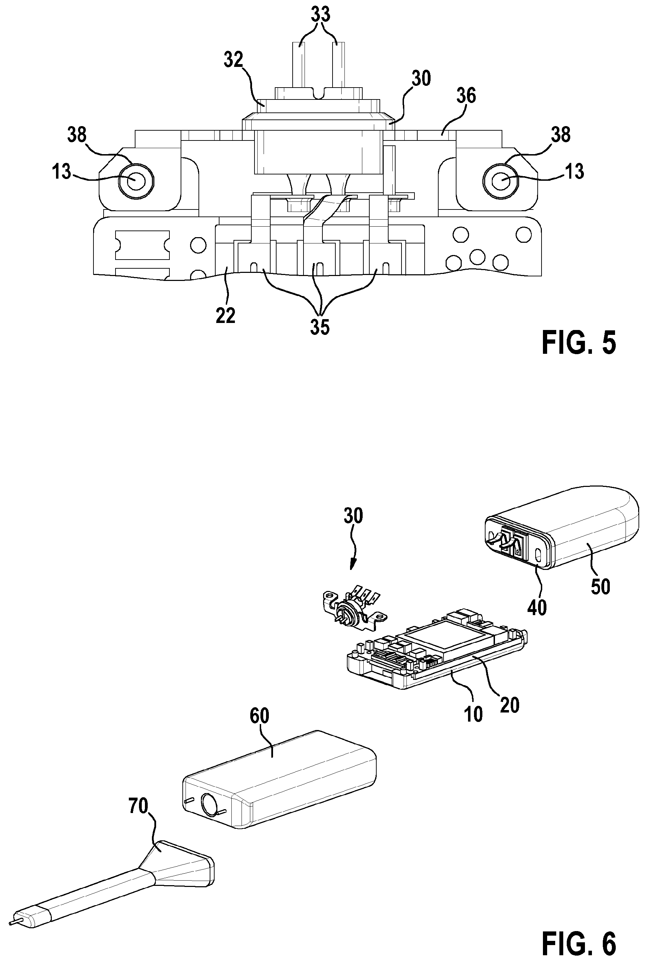

In Fig. 3 a feedthrough component 30 is depicted comprising an electrical insulating sealing body 32 realizing the hermetic sealing of the interior of a sleeve type circuit housing 60 (see Fig. 6) when accommodated within the housing 60. The feedthrough component 30 further comprises contact pins 33 for providing the electrical contact to a plug 70 (connector element, see Fig. 6). On the front side of the sealing body opposite the contact pins 33 the feedthrough component 30 provides a tab array of three tabs 35 which are adapted to provide the electrical contact to the electrical circuit unit 20. The feedthrough component 30 further comprises a bracket 36 with two L-shaped arms, wherein when assembled with the support 10 and the electrical circuit unit 20 a first arm (36a) of the L is situated perpendicular to the extension (see arrows 14 in Fig. 1) of the support 10 and the electrical circuit unit 20 and second arm 36b of the L runs parallel to this extension. In each arm 36b a circular through going opening 38 is provided. The bracket 36 of the feedthrough component 30 not only facilitates the mechanical connection to the support 10 and the electrical circuit unit 20 but also forms a weld shield. One preferred material of the bracket 36 is a metallic material, for example titanium. Other suitable materials - also suitable non-metallic materials - are possible. These material/materials/material composition may be incorporated in the bracket, plated, coated on the bracket or otherwise applied to the bracket. In one exemplary embodiment these materials or material compositions are solely located on the bracket location, which is situated between the vent hole in the medical device housing and the electrical circuit unit. Further materials of the feedthrough components are commonly known like Niobium as material for pins 33, Titanium as material for flange or sealing body 32 and ceramics or gold solder for hermetically tightening the feedthrough.

As depicted in Figs. 4 and 5 the feedthrough component 30 is assembled with the support 10 and the electrical circuit unit 20 such that each opening 38 of the bracket 36 of the feedthrough component 30 is placed over one of the staking pins 13 establishing a form-fit connection. Thereby, the feedthrough component 30 is properly aligned and mechanically attached relative to the support 10 and the electrical circuit unit 20. The tabs 35 form an electric connection to a respective contact unit (interconnect weld ribbon) 22 of the electrical circuit unit 20.

During manufacturing of the implantable medical device in the first step a support 10 is provided as depicted in Fig. 1. After that, the electrical circuit unit 20 is fixedly connected for example by an adhesive or by solderable pads 17A to the support 10. In a preferred embodiment an antenna is attached to the support 10 prior fixing the electrical circuit unit 20 to the support 10. Then, the feedthrough component 30 is mechanically connected and aligned to the support 10 using the two staking pins 13 of the support 10 and the two through going openings 38 of the feedthrough component 30. After that the staking pins 13 are molded over the bracket 36 by means of a heat staking process. This process uses a heated metal tip that has the negative shape of the stake (final shape of the overmolded pin). The subassembly is located in a tooling nest that provides support and alignment of the subassembly in the process. The heated metal tip is being pressed on the staking pin with a controlled speed and travel lengh, while the pin is melting and molded into the shape of the metal tip. At the end, the metal tip releases and a strong mechanical attachment and reliable alignment of the feedthrough component 30 relative to the support 10 and the electrical circuit unit 20 is established. Then, the tabs 35 are electrically connected to the contact unit 22 of the electrical circuit unit 20.

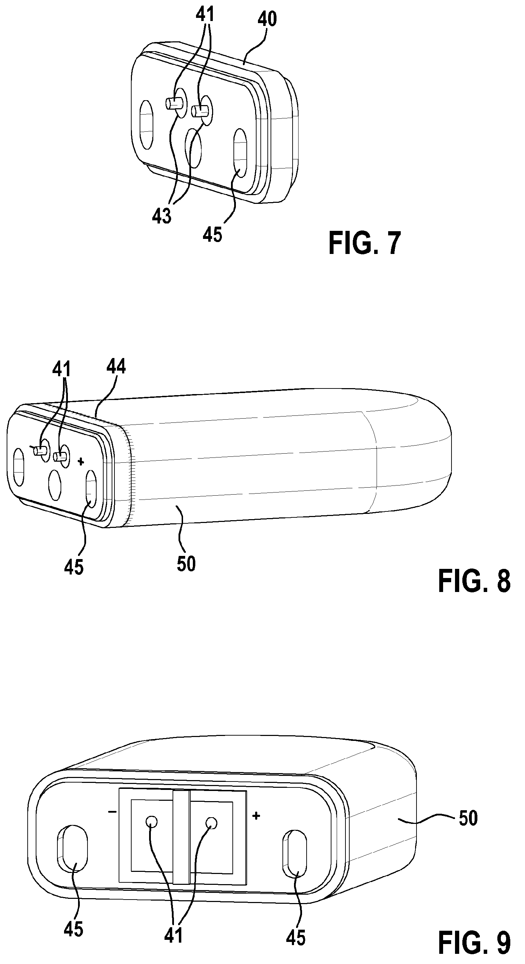

The assembly containing the power source (e.g. chemical battery, capacitive storage device and so forth, not shown) is manufactured prior the production of the circuit assembly or parallel. Therefore, a flange 40 as shown in Fig. 7 is provided forming the cover of a battery housing 50. The flange 40 comprises the electric connections with the power source forming connecting pins 41 at the outer side of the flange 40. Each connecting pin 41 is surrounded by a glass feedthrough 43 in order to isolate the electric conducting material of the pin 41 with regard to the flange 40.

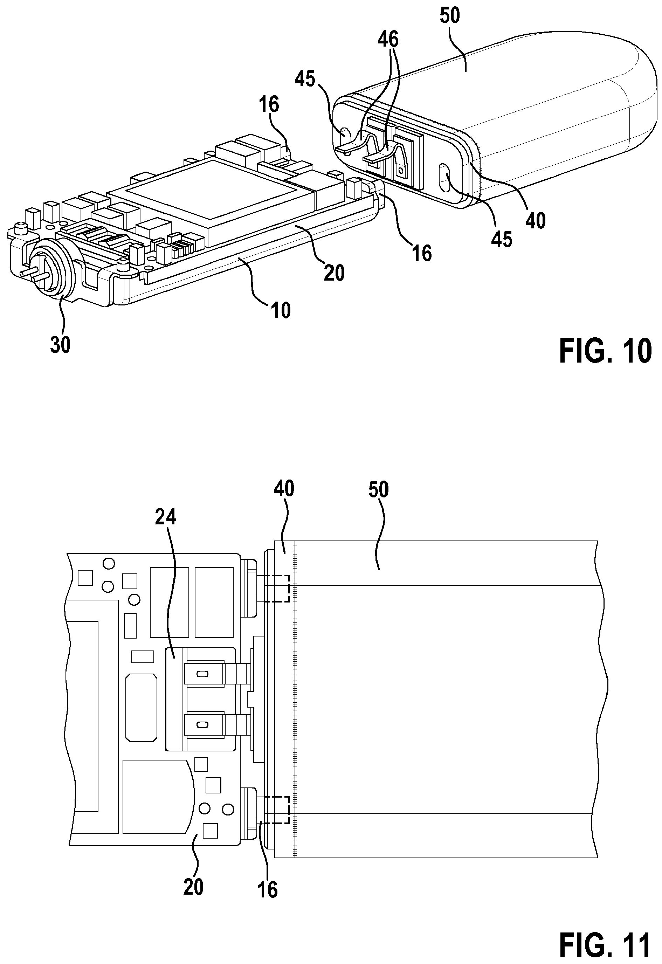

After placement of the power source within the battery housing 50 the flange 40 is connected to the battery housing by laser welding (see weld seam 44 in Fig. 8) such that the flange 40 forms a cover of the battery housing 50 and hermetically seals the battery housing 50. The flange 40 connects the power source with the assembly containing electrical circuit unit 20. The electric contact to the electrical circuit unit 20 is provided by pins 41 as shown in Fig. 7 or alternatively by a tab array. Accordingly, the electrical circuit unit 20 may have a tape 24 for connection with the tabs 46 of the tab array (see Figs. 10, 11).

The flange 40 provides a distinctive and a tolerance compensating plug connection of the electrical circuit unit 20 with the antenna. There is only one predefined possibility (form-fit) to connect (mechanically) the electrical circuit unit 20 and the battery housing 50 comprising the flange 40 and the power source. The flange 40 further has the advantage that a single-axis assembly is possible. For form fitting the flange 40 provides openings 45 for accommodation of the battery tabs 16 of the support 10 after the assembly of flange 40 with the battery housing 50 is produced (see Figs. 10 and 11). After mechanical connection of the battery assembly with the assembly shown in Figs. 4 and 5 the tabs 46 are electrically connected with the tape 24 of the electrical circuit unit 20.

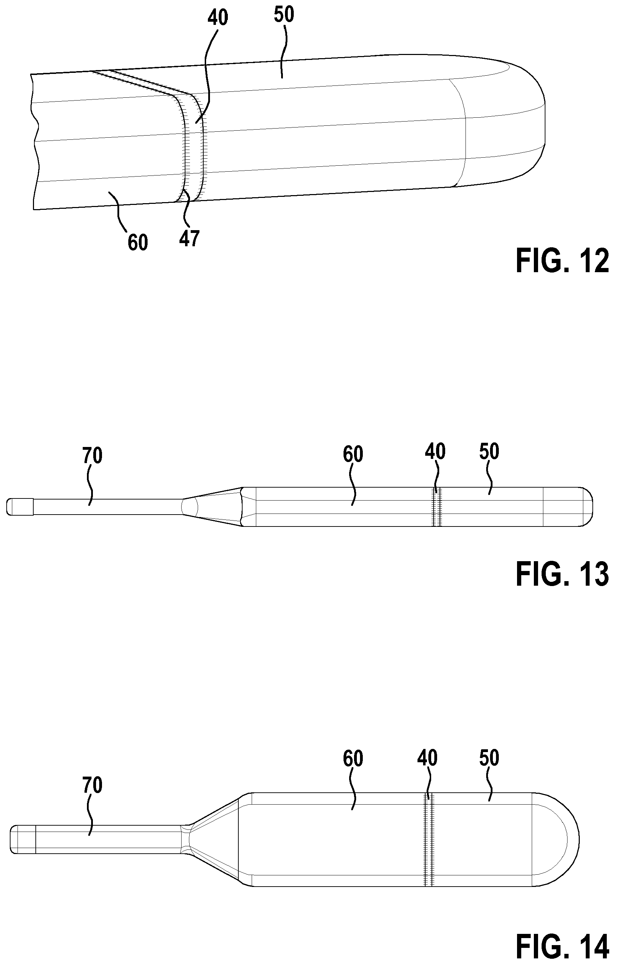

Then, the long, sleeve-type circuit housing 60 (can) is placed over the assembly comprising the support 10, the electrical circuit unit 20 and the feedthrough component 30. The circuit housing 60 is connected with the flange 40 by laser welding (see Fig. 12, weld seam 47). Thereby the circuit housing 60 is hermetically sealed. Finally, the plug 70 is mechanically connected with the circuit housing 60 at the side opposite to the battery housing 50 and flange 40 and electrically connected with the contact pins 33 of the feedthrough component 30. The completed implantable medical device is shown in Figs. 13 and 14 in two different views.

The inventive medical device with the support 10 has the advantage that it is easy to produce with less number of parts and provides the possibility to use high density interconnect flex substrates for the electronic circuit unit 20 allowing low device profile. Further, the form-fit connection between support 10 and the feedthrough component 30 utilizes the space in the device that would otherwise have been empty owing to the need for clearance between the electrical circuit unit 20 and the circuit housing 60 and is, therefore, space neutral. The self-alignment of the feedthrough component 30 relative to the electrical circuit unit 20 and the support 10 along the long axis of the device leads to a simple assembly with a low number of process steps. Further, a strong mechanical attachment and reliable alignment is provided.

The battery flange 40 has the advantage that the battery housing 50 and the circuit housing 60 may be closed by laser welding. The flange further serves as a tolerance compensation relative to the longitudinal axis of the housing and makes a single-axis assembly possible. Thereby, the device may be minimized and provides the possibility to choose the material of the electronic circuit unit independently from the mechanical structure of the device.

### Reference numbers

- 10: support
- 11: nose
- 13: staking pin
- 14: arrows showing the extension of the support and the electrical circuit unit 20
- 15: stiffening rib
- 16: battery tab
- 17: interposer or printed circuit board (PCB) construction
- 17A: solderable pad
- 18: contact element
- 19: via
- 20: electrical circuit unit
- 22: contact unit
- 24: tape
- 30: feedthrough component
- 32: sealing body
- 33: contact pin
- 35: tab
- 36: bracket
- 36a: first arm of the L-form of the bracket 36
- 36b: second arm of the L-form of the bracket 36
- 38: through going opening
- 40: flange
- 41: connecting pin
- 43: glass feedthrough
- 44: weld seam
- 45: opening
- 46: tab
- 47: weld seam
- 50: battery housing
- 60: circuit housing
- 70: plug

## Claims

1. An implantable medical device comprising an housing (60), an electrical circuit unit (20), and a feedthrough component (30), which is electrically connected to at least one contact of the electrical circuit unit, wherein the device further comprises a support (10) fixedly connected to the electrical circuit unit (20), **characterized in that** the support (10) provides at least one fixture element (13) adapted for a mechanical fixing of the feedthrough component (30) relative to the electrical circuit unit (20) within the housing (60).

2. The implantable medical device according to claim 1, wherein the at least one fixture element is a form-fit element, for example a staking pin (13), cooperating with a respective form-fit element, for example a through going opening (38), of a bracket (36) of the feedthrough component (30).

3. The implantable medical device according to any of the preceding claims, wherein the fixture element (13) is accommodated in the clearance between the electrical circuit unit (20) and the housing (60) parallel to the feedthrough component (30).

4. The implantable medical device according to any of the preceding claims, wherein the support (10) is formed as a frame and preferably adapted to carry a passive electronic module such as an antenna.

5. The implantable medical device according to any of the claims 2 to 4, wherein the bracket (36) of the feedthrough component comprises an L-form, wherein a first arm (36a) of the L runs perpendicular to the extension of the electrical circuit unit (20) and a second arm (36b) of the L comprises the form-fit element (38) and runs parallel to the extension of the electrical circuit unit (20).

6. The implantable medical device according to any of the preceding claims, wherein the electrical circuit unit (20) comprises an interconnect weld ribbon and the feedthrough component (30) comprises at least one tab (35) for electrical connection.

7. The implantable medical device according to any of the claims 1 to 5, wherein the electrical circuit unit (20) comprises a flexarm extension for electrical connection with the feedthrough component (30).

8. A manufacturing method for an implantable medical device comprising a housing (60), an electrical circuit unit (20), a feedthrough component (30) and a support (10) comprising the following steps:
- fixedly connecting of the electrical circuit unit (20) with the support (10),
- mechanical fixing of the feedthrough component (30) relative to the electrical circuit unit (20) by means of at least one fixture element (13) provided at the support (10),
- forming an electrical connection of the feedthrough component (30) with at least one contact of the electrical circuit unit (20),
- placing the assembly comprising the support (10) with the electrical circuit unit (20) and the feedthrough component (30) within the housing (60) and hermetically sealing the housing (60).

9. The method according to claim 8, wherein for mechanical fixing of the feedthrough component (30) by means of the at least one fixture element (13) provided at the support (10) the fixture element (13) of the support (10) is form-fit connected to a corresponding fixture element (38) at the feedthrough component (30) and preferably thereafter fixed by a material engagement.

10. The method according to any of the claims 8 to 9, wherein prior to the connection of the electrical circuit unit (20) with the support (10) a passive electronic module, preferably an antenna, is attached to the support (10).
